# FASCICULE DE BREVET EUROPEEN

(11) **EP 1 354 873 B1**
(45) Date de publication et mention de la délivrance du brevet: **14.07.2004**
(21) Numéro de dépôt: 03290383.3
(22) Date de dépôt: 17.02.2003
(51) Int. Cl.: C07D 209/42, A61K 31/404, A61P 9/12

(54) **Sel de périndopril et les compositions pharmaceutiques qui le contiennent**
Perindoprilsalz und dieses enthaltende Arzneimittel
Salt of perindopril and pharmaceutical compositions containing it

(30) Priorité: 18.04.2002 FR 0204847
(43) Date de publication de la demande: 22.10.2003
(73) Titulaire: Les Laboratoires Servier, 92415 Courbevoie Cedex (FR)
(72) Inventeur: Damien, Gérard, 45000 Orleans (FR); Lefoulon, François, 45000 Orleans (FR); Marchand, Bernard, 78480 Verneuil sur Seine (FR)

(56) Documents cités:
- WO-A-01/87835

## Description

La présente invention concerne un nouveau sel de périndopril et les compositions pharmaceutiques qui le contiennent. Le périndopril ou acide (2S)-2-[(1S)-carbéthoxybutylamino]-1-oxopropyl-(2S,3aS,7aS)perhydroindole carboxylique, inhibiteur de l'enzyme de conversion de l'angiotensine I (IEC) est un composé connu plus particulièrement pour le traitement de l'hypertension artérielle et de l'insuffisance cardiaque.

Le périndopril a été précédemment décrit dans le brevet EP 0 049 658. Dans ce brevet européen, de manière classique, il est mentionné que les composés de l'invention peuvent se présenter sous forme de sels d'addition avec une base ou un acide minéral ou organique pharmaceutiquement acceptable. Les composés décrits dans ce brevet sont sous forme non salifiée et lorsque des sels d'addition à une base ou à un acide pharmaceutiquement acceptable sont exemplifiés, on trouve principalement le sel de sodium ou le maléate.

Or il s'est avéré très difficile lors du développement de ce produit de trouver un sel pharmaceutiquement acceptable présentant non seulement une bonne biodisponibilité, mais également une stabilité suffisante pour être compatible avec la préparation et la conservation de compositions pharmaceutiques.

Dans les études qui ont été menées à l'origine sur ce produit, le sel de *tert*-butylamine de périndopril s'est avéré présenter des qualités suffisantes pour le développement du produit et c'est ce sel de tert-butylamine de périndopril qui est actuellement commercialisé.

La forme non salifiée a été étudiée ainsi que le maléate et le sel de sodium. Lors d'études de stabilité à la température et à l'humidité, il a été montré que le sel de sodium n'était pas manipulable car il se transforme immédiatement en huile au contact de l'atmosphère, la forme non salifiée et le maléate se dégradant quant à eux rapidement dans ces conditions (25 à 30 % de produit dégradé environ en 8 jours à 50°C).

Ainsi, seul le sel de *tert*-butylamine présentait la meilleure stabilité en comparaison des autres formes étudiées. Cependant, compte-tenu de la fragilité intrinsèque du périndopril, le sel de *tert*-butylamine ne permettait pas de résoudre complètement les problèmes de stabilité du produit à la chaleur et à l'humidité. En effet, pour sa commercialisation, les comprimés sel de *tert*-butylamine de périndopril doivent, dans certains pays, être protégés par des suremballages. D'autre part, même pour les pays à climat tempéré, cette instabilité n'a pas permis d'obtenir une date de péremption des comprimés supérieure à 2 ans. Enfin ces comprimés doivent être commercialisés avec la mention "à conserver à une température inférieure ou égale à 30 degrés".

Ces contraintes sont bien entendu lourdes, notamment en terme d'organisation et de coût, et il est apparu particulièrement intéressant de tenter de mettre au point un nouveau sel de périndopril afin de diminuer les contraintes dues au sel de *tert*-butylamine.

De nombreux sels ont été étudiés et comme indiqué précédemment, les sels classiquement utilisés dans le domaine pharmaceutique se sont avérés inutilisables.

En revanche, et de manière surprenante, il a été trouvé que le sel d'arginine du périndopril, outre le fait qu'il soit nouveau, présente des avantages totalement inattendus par rapport à tous les autres sels étudiés et plus particulièrement par rapport au sel de *tert*-butylamine du périndopri 1.

La présente invention concerne donc le sel d'arginine de périndopril, ses hydrates ainsi que les compositions pharmaceutiques qui le contiennent.

Le sel d'arginine sera préférentiellement le sel d'argine naturelle ou sel de L-arginine.

Les compositions pharmaceutiques selon l'invention contiennent donc du sel d'arginine de périndopril avec un ou plusieurs excipients, non toxiques, pharmaceutiquement acceptables et appropriés.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

A titre préférentiel, les compositions pharmaceutiques selon l'invention seront des comprimés à libération immédiate.

La posologie utile varie selon l'âge et le poids du patient, la nature et la sévérité de l'affection ainsi que la voie d'administration. Celle-ci peut être orale, nasale, rectale ou parentérale.

Dans les compositions selon l'invention, la quantité de sel d'arginine est comprise entre 0,2 et 10 mg, préférentiellement entre 1 et 10 mg. Ces compositions pharmaceutiques sont utiles pour le traitement de l'hypertension et de l'insuffisance cardiaque.

Les caractéristiques essentielles de ce sel sont une très grande stabilité à la chaleur et à l'humidité comparativement au sel de *tert*-butylamine.

En effet, des études de stabilité réalisées sur le long terme dans des conditions de températures et d'humidité bien précises ont conduit au résultats mentionnés dans le tableau ci-dessous.

Dans cette étude, le périndopril a été dosé par chromatographie liquide haute pression en phase inverse en utilisant comme éluant une phase aqueuse (contenant de l'heptane sulfonate de sodium et dont le pH est égal à 2) et de l'acétonitrile (67/33). Le produit a été détecté par UV (215 nm).

Cette étude a été réalisée avec des comprimés à libération immédiate contenant soit 2,4 mg de sel d'arginine de périndopril, soit 2,0 mg de sel de *tert*-butylamine de périndopril (ces deux comprimés contiennent chacun 1,7 mg de périndopril). Le dosage des comprimés est effectué après 6 mois de mise en stabilité des comprimés, à différentes températures et à différents pourcentages d'humidité relative (HR).

Le sel d'argine utilisé dans cette étude est le sel de L-arginine. Il a été préparé selon une méthode classique de salification de la chimie organique.

| **Conditions 6 mois** | **Sel de *tert*-butylamine de périndopril Pourcentage restant (%)** | **Sel d'arginine de périndopril Pourcentage restant (%)** |
|---|---|---|
| 25° C 60% HR | 101,0 | 99,5 |
| 30°C 60% HR | 94,4 | 98,1 |
| 40°C 75 % HR | 67,2 | 98,6 |

Les résultats présentés dans le tableau ci-dessus montrent donc de manière extrêmement claire la très grande stabilité du sel d'arginine par rapport au sel de *tert*-butylamine. En effet, après 6 mois, le sel d'arginine n'est pratiquement pas dégradé alors que le sel de *tert*-butylamine présente un taux de dégradation d'environ 33 %.

Ces résultats sont totalement inattendus et ne pouvaient être déduits ou suggérés de l'enseignement de la littérature sur ce produit.

Ils nous permettent d'envisager des contraintes moins lourdes en matière de conditionnement des compositions pharmaceutiques et d'autre part d'obtenir une durée de péremption de nos compositions pharmaceutiques au moins égales à trois ans.

## Revendications

1. Sel d'arginine de périndopril ainsi que ses hydrates.

2. Composition pharmaceutique contenant comme principe actif le sel d'arginine de périndopril ainsi que ses hydrates en combinaison avec un ou plusieurs excipients pharmaceutiquement acceptables.

3. Composition pharmaceutique selon la revendication 2 **caractérisé en ce qu'**elle **se** présente sous forme de comprimé à libération immédiate.

4. Composition pharmaceutique selon l'une quelconque des revendications 2 ou 3 **caractérisée en ce qu'**elle contient de 0,2 à 10 mg de sel d'arginine de périndopril.

5. Composition pharmaceutique selon l'une quelconque des revendications 2 à 4 utile pour la préparation d'un médicament destiné au traitement de l'hypertension et de l'insuffisance cardiaque.

## Patentansprüche

1. Argininsalz von Perindopril und seine Hydrate.

2. Pharmazeutische Zubereitung, enthaltend als Wirkstoff ein Argininsalz von Perindopril sowie seine Hydrate in Kombination mit einem oder mehreren pharmazeutisch annehmbaren Trägermaterialien.

3. Pharmazeutische Zubereitung nach Anspruch 2, **dadurch gekennzeichnet, daß** sie in Form einer Tablette mit sofortiger Wirkstofffreisetzung vorliegt.

4. Pharmazeutische Zubereitung nach einem der Ansprüche 2 oder 3, **dadurch gekennzeichnet, daß** sie 0,2 bis 10 mg des Argininsalzes von Perindopril enthält.

5. Pharmazeutische Zubereitung nach einem der Ansprüche 2 bis 4 für die Herstellung eines Arzneimittels zur Behandlung der Hypertension und der Herzinsuffizienz.

## Claims

1. The arginine salt of perindopril and hydrates thereof.

2. Pharmaceutical composition comprising, as active ingredient, the arginine salt of perindopril, or a hydrate thereof, in combination with one or more pharmaceutically acceptable excipients.

3. Pharmaceutical composition according to claim 2, **characterised in that** it is presented in the form of an immediate-release tablet.

4. Pharmaceutical composition according to either claim 2 or claim 3, **characterised in that** it contains from 0.2 to 10 mg of the arginine salt of perindopril.

5. Pharmaceutical composition according to any one of claims 2 to 4 for use in the preparation of a medicament intended for treating hypertension and heart failure.
